# EUROPÄISCHE PATENTSCHRIFT

(11) **EP 0 554 590 B1**
(45) Veröffentlichungstag und Bekanntmachung des Hinweises auf die Patenterteilung: **10.01.1996**
(21) Anmeldenummer: 92250277.8
(22) Anmeldetag: 30.09.1992
(51) Int. Cl.: C07C 69/675, C07C 67/31, C07C 69/708, C07C 229/22, C07C 231/12, C07C 227/28, C07C 235/10, C07C 229/26

(54) **Verfahren zur Herstellung hydroxylierter Fettsäureverbindungen**
Process for the preparation of hydroxylated fatty acids
Procédé de préparation de composés d'acides gras hydroxylés

(30) Priorität: 04.02.1992 DE 4203077
(43) Veröffentlichungstag der Anmeldung: 11.08.1993
(73) Patentinhaber: HARBURGER FETTCHEMIE BRINCKMAN & MERGELL GmbH, D-21079 Hamburg (DE)
(72) Erfinder: Hellbardt, Siegfried, Dr., W-2100 Hamburg 90 (DE); Zech, Walter H., W-2000 Hamburg 73 (DE); Schlandt, Klaus, W-2105 Seevetal 2 (DE)
(74) Vertreter: UEXKÜLL & STOLBERG

(56) Entgegenhaltungen:
- EP-A- 0 083 970
- EP-A- 0 340 587
- DE-C- 4 125 031
- FR-A- 1 349 807

## Beschreibung

Die Erfindung betrifft ein Verfahren zur Herstellung hydroxylierter Fettsäureverbindungen durch Oxidation epoxidierbarer Fettsäureverbindungen unter Ausbildung von Oxiranringen und säurekatalysierte Umwandlung der epoxidierten Fettsäureverbindungen.

Der Begriff Fettsäureverbindung bedeutet vorliegend sowohl die Fettsäuren selbst als auch Fettsäureverbindungen wie Amide und Ester, beispielsweise die Fettsäuretriglyceride, welche pflanzliche und tierische Öle und Fette umfassen. Die in diesen Triglyceriden oder anderen Fettsäureverbindungen gebundenen Fettsäuren können frei von Doppelbindungen, d.h. gesättigte Fettsäuren sein oder eine oder mehrere Doppelbindungen enthalten, d.h. ungesättigte Fettsäuren sein. Die natürlich vorkommenden Triglyceride enthalten im allgemeinen sowohl gesättigte als auch einfach und mehrfach ungesättigte Fettsäuren. Einige Fettsäuren natürlich vorkommender Öle weisen auch Hydroxylgruppen in den Fettsäureresten auf. Von den industriell in größerem Umfang genutzten Ölen hat nur das Rizinusöl wegen der dominanten Fettsäure, nämlich die Rizinolsäure, sowohl eine Doppelbindung als auch eine Hydroxylgruppe je Molekül.

Fettsäureverbindungen der obigen Definition dienen als Ausgangsmaterial zur Erzeugung einer Vielzahl technisch wichtiger Produkte, wobei Fettsäureverbindungen mit Doppelbindungen und/oder Hydroxylgruppen von besonderem Interesse sind, da sich aus diesen zahlreiche Polymere, insbesondere Kunststoffe herstellen lassen. Insbesondere kann man ausgehend von Fettsäureverbindungen, die eine oder mehrere Alkoholgruppen je Triglyceridmolekül enthalten, durch Umsetzung mit entsprechenden reaktiven Verbindungen wie z.B. Diisocyanaten zu polymeren Strukturen gelangen.

Anwendungsbereiche für aus hydroxylierten Fettsäureverbindungen erhaltene technische Produkte sind allgemein technische Hilfsstoffe, Kunststoffe wie Polyurethanamide, Polyesteramide, Polyesterurethane, Tenside, Schmierstoffe, Polyester oder Polyamide.

Von den aus nachwachsenden Rohstoffen gewonnenen Ölen sind die ölsäurereichen Öle wie Sonnenblumenöl und Euphorbia Lathyris-Öl von besonderem Interesse.

Fettsäuren und Fettsäureverbindungen mit zwei oder mehr Hydroxylgruppen je Fettsäurerest sind bekannt. So beschreiben T.M. Luong, H. Schriftman und D. Swern in J. Am. Oil Chem. Soc. 44, 316-320 (1967) die Herstellung von 1,2-Glykolen ausgehend von bestimmten wasserunlöslichen monoungesättigten Fetten und Fettsäurederivaten wie Ölsäure, Methyloleat, Oleylalkohol, Olivenöl oder Rizinusöl mittels eines Wasserstoffperoxid-Wolframsäure-Oxydationssystems bei pH-Werten von 0 bis 1 und anschließender Hydratisierung der nicht zu isolierenden Epoxidzwischenprodukte.

Bekannt ist ferner aus US 2 443 280 ein Verfahren zum Herstellen mehrwertiger Alkohole, bei welchen sich die Hydroxylgruppen an benachbarten Kohlenstoffatomen befinden, wobei diese α-Glykole durch Hydroxylierung monoungesättigter aliphatischer Verbindungen hergestellt werden, und zwar durch Umsetzung mit einer Mischung von Wasserstoffperoxid und Essigsäure sowie katalytischen Mengen einer starken Säure wie Schwefelsäure. Diese Umsetzung erfolgt bei mäßigen Temperaturen und verläuft vermutlich über eine Epoxidverbindung, die dann hydrolysiert wird.

Aus der japanischen Patentanmeldung Kokai Nr. 64-000195 sind als Schneidöle hydroxylierte Produkte von Fetten und Ölen bekannt, die gemäß der japanischen Patentanmeldung Kokai Nr. 63-13993 hergestellt werden. Bei diesem Verfahren werden tierische und pflanzliche Öle und ungesättigte Fette epoxidiert und anschließend ein Teil der Epoxidgruppen unter Bildung von Hydroxylgruppen geöffnet.

Aus C.K. Ingold, Structure and Mechanism in Organic Chemistry, Cornell University Press, New York 1953; J.L. Bromsted, J.Amer. Chem.Soc. 51, 428 (1929) und H.J. Lichtenstein, Trans. Faraday Soc. 44, 905 (1948) ist ein Verfahren einer säurekatalysierten Öffnung des Epoxidrings durch Anlagerung von Wasser bekannt, das beispielsweise bei der Hydroxylierung von Ethylenoxid zu Ethylenglykol angewandt wird.

Aus der oben erwähnten J.Am. Oil Chem.Soc.44, 316-320 (1967) ist aber gleichfalls zu entnehmen, daß bei Triglyceriden mit größerer Kettenlänge der Fettsäuren und mittelständiger Position der Oxirangruppe bei denselben die säurekatalysierte Ringöffnung unter Bildung von 1,2-Glykolen schwieriger und damit uninteressant wird.

Es ist die Aufgabe der Erfindung, ein Verfahren zur Herstellung hydroxylierter Fettsäureverbindungen verfügbar zu machen, die mittelständige Hydroxylgruppen im Fettsäurerest aufweisen, welches wirtschaftlich und umweltfreundlich verläuft. Auch ist es die Aufgabe der Erfindung, hydroxylierte Fettsäureverbindungen, vor allem Triglyceride, mit zwei oder mehreren in der Regel im Bereich der C-Atome 9 bis 16 stehenden Hydroxylgruppen verfügbar zu machen. Schließlich ist es die Aufgabe der Erfindung, hydroxylierte Fettsäureverbindungen, vor allem Triglyceride, mit einer oder mehreren in der Regel im Bereich der C-Atome 9 bis 16 stehenden Hydroxylgruppen und diesen Hydroxylgruppen benachbarten Sustituenten verfügbar zu machen.

Zur Lösung dieser Aufgabe wird ein Verfahren gemäß dem kennzeichnenden Teil des Hauptanspruchs vorgeschlagen. Die Unteransprüche beinhalten bevorzugte Ausbildungsweisen des Verfahrens.

Es ist angesichts des Standes der Technik völlig überraschend, daß man ausgehend von den entsprechenden zu epoxidierenden Fettsäureverbindungen wie z.B. epoxidierten Triglyceriden oder anderen epoxidierten Fettsäureverbindungen eine säurekatalysierte Öffnung des Epoxidrings unter Ausbildung von zwei Hydroxylgruppen je Epoxidring oder von einer Hydroxylgruppe und einem benachbarten Sustituenten je Epoxidring fast quantitativ erzielen kann, wenn man als Katalysator ein mit unterschiedlichen mineralischen oder organischen Säuren wie z.B. HCl, H₃PO₄, H₂SO₄ säureaktiviertes Trägermaterial mit großer Oberfläche und einem großen Adsorptionsvermögen verwendet wie Bleicherde oder aber auch Aktivkohlen und/oder oberflächenaktive Tonerden und/oder Silikate.

Die säurekatalysierte Ringöffnung des Oxiranrings epoxidierter Fettsäureverbindungen gemäß Erfindung verläuft praktisch vollständig. Die Reaktion benötigt nur sehr geringe Katalysatormengen und verläuft glatt. Deshalb ist das erfindungsgemäße Verfahren auch äußerst umweltfreundlich, weil praktisch keine Entsorgungsprobleme verursachende Nebenprodukte entstehen.

Zur Durchführung des Verfahrens wird vorzugsweise von gereinigten pflanzlichen und/oder tierischen Fetten und/oder Ölen und/oder Fettsäureverbindungen mit unterschiedlichen Anteilen an einfach und/oder mehrfach ungesättigten Fettsäuren, insbesondere von Fettsäuretriglyceriden, die in an sich bekannter Weise epoxidierbar sind, ausgegangen. Nach der Epoxidierung werden die teilweise oder vollständig epoxidierten Fettsäureverbindungen zweckmäßig mit 0,001 % oder 0,01 % oder mehr, vorzugsweise 0,01 bis 0,1 % des säureaktivierten Katalysators wie einer säureaktivierten Bleicherde und der für die Umwandlung der Oxirangruppen zu einer OH-Gruppe und einem dieser OH-Gruppe benachbarten Substituenten erforderlichen Menge an Wasser oder an aktiven Wasserstoff enthaltenden Verbindung zur Reaktion gebracht. Die Reaktion wird vorteilhaft in einem geschlossenen System unter Rühren bei Temperaturen zwischen 80 und 200°C, vorzugsweise bei 120 bis 200°C durchgeführt.

Die Hydroxylierung setzt innerhalb dieses Temperaturbereichs spontan ein und läuft dann innerhalb kürzester Zeit zu mehr als 80 % ab. Zur Umwandlung des Restoxirans auf einen Oxirangehalt von etwa 0,1 % wird das Reaktionsgemisch bei der erreichten Temperatur etwa 1 Stunde gehalten.

Die Hydroxylierung eines epoxiderten Triglycerids mit Wasser oder mit aktiven Wasserstoff enthaltenden Verbindungen läuft nach folgendem Reaktionsschema ab:

### Epoxidiertes Triglycerid:

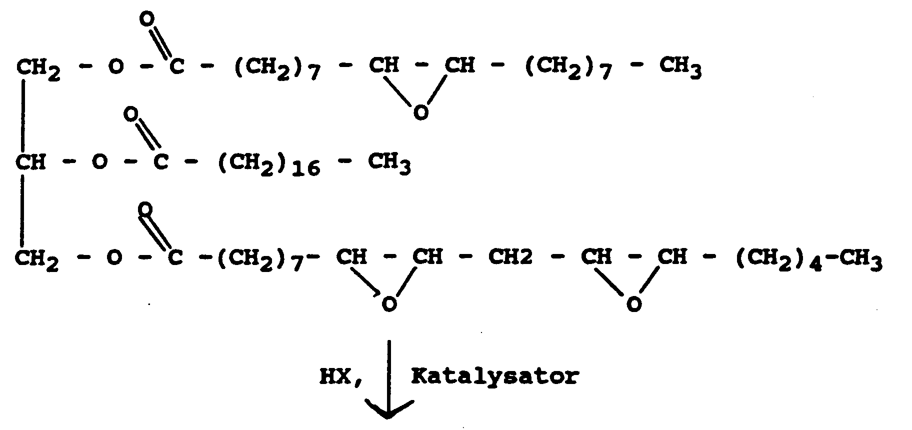

### Hydroxyliertes Triglycerid:

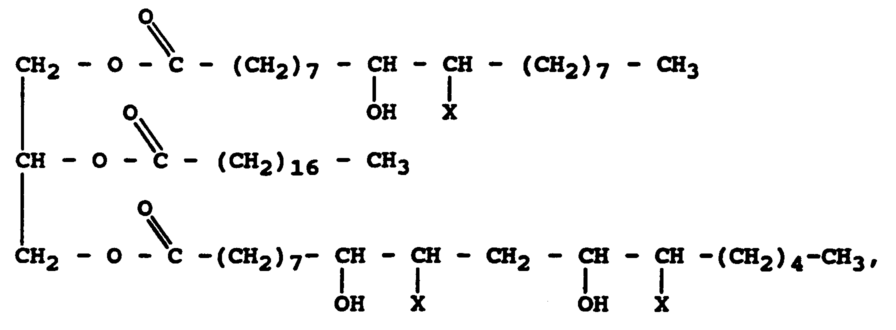

wobei
- **X**: = - OH bzw. - O - R bzw. - O - R - OH
- O - Ar 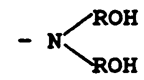 bzw. - HN - ROH 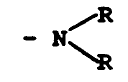 bzw. - HN - R
- S - R 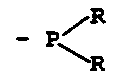 bzw. - HP - R 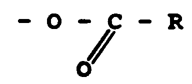 - F; Cl; Br; J

Die folgenden Beispiele erläutern die Erfindung:

### Beispiel 1: Hydroxylierung von epoxidiertem Sojaöl

1000 g epoxidiertes Sojaöl mit einem Oxirangehalt von 6,65 % wurden mit 100 g Wasser und 1 g einer mit Salzsäure aktivierten Tonerde vom Montmorillonit-Typ in einem Autoklaven unter Rühren aufgeheizt. Ab ca. 130°C setzte die Hydratisierung der Oxirangruppen unter Bildung von Hydroxylgruppen ein und führte nach Erreichen einer Temperatur von ca. 180°C innerhalb weniger Minuten zu einem Abbau des Oxirangehalts auf ca. 1 %. Zum vollständigen Abbau dieses Rest-Oxirans wurde das Reaktionsgemisch noch eine weitere Stunde unter Rühren bei ca. 180°C gehalten.

Das derart gewonnene hydroxylierte Sojaöl besaß folgende Kennzahlen: SZ 5,4; JZ 11; Oxirangehalt 0,02 %; OHZ 354; Viskosität bei 25°C 29.000 mPa.s.

### Beispiel 2: Hydroxylierung von epoxidiertem Öl von Euphorbialathyris (Euphorbiaöl)

1000 g epoxidiertes Euphorbiaöl mit ca. 85 % 9,10-Epoxystearinsäure und einem Oxirangehalt von 4,58 % wurden mit 100 g Wasser und 1 g einer mit Salzsäure aktivierten Tonerde vom Montmorillonit-Typ in einem Autoklaven unter Rühren aufgeheizt. Bei ca. 150°C setzte die Hydratisierung der Oxirangruppen unter Bildung von Hydroxylgruppen ein und führte bei Anstieg der Temperatur auf ca. 185°C innerhalb weniger Minuten zu einem Abbau des Oxirangehaltes auf ca. 0,8 %. Zum vollständigen Abbau dieses Rest-Oxirans wurde das Reaktionsgemisch noch eine weitere Stunde unter Rühren bei ca. 185°C gehalten.

Das so gewonnene hydroxylierte Euphorbiaöl besaß folgende Kennzahlen: SZ 5,0; JZ 7,2; Oxirangehalt 0,1 %; OHZ 287; Erweichungstemperatur ca. 55°C.

### Beispiel 3: Hydroxylierung eines epoxidierten Rübölfettsäure-2-ethylhexylesters

1000 g eines epoxidierten Rübölfettsäure-2-ethylhexylesters mit den Kennzahlen SZ 0,15; JZ 2,5 und einem Oxirangehalt von 4,3 % wurden mit 100 g Wasser und 1 g einer mit Salzsäure aktivierten Tonerde vom Montmorillonit-Typ unter den in Beispiel 1 genannten Bedingungen umgesetzt. Nach 1-stündiger Reaktionszeit war der Oxirangehalt total abgebaut.

Der auf diese Weise erhaltene hydroxylierte Ester besaß folgende Kennzahlen: SZ 18,5; JZ 20,6; Oxirangehalt 0 %; OHZ 190; Viskosität bei 25°C 162 mPa.s.

### Beispiel 4: Hydroxylierung eines Sonnenblumenölfettsäure-diamids

Durch Amidierung von destillierter Sonnenblumenölfettsäure (Kennzahlen: Linolsäuregehalt 61,4 %; SZ 201; JZ 139) mit Ethylendiamin wurde nach bekanntem Verfahren ein Diamid mit folgenden Kennzahlen hergestellt: SZ 12,3; AZ 15,0; JZ 137; Erweichungstemperatur ca. 80°C. Das in bekannter Weise mit Perameisensäure epoxidierte Diamid wies folgende Kennzahlen auf: SZ 6,6; JZ 35,8; Oxirangehalt 2,7 %; OHZ 154. Bei anschließender Hydroxylierung des epoxidierten Diamids unter den in Beispiel 1 genannten Bedingungen und Einhaltung der dort gewählten Mengenverhältnisse wurde ein Hydroxyfettsäure-Diamid mit folgenden Kennzahlen erhalten:
SZ 5,9; JZ 30,2; Oxirangehalt 0,1 %; OHZ 395; Erweichungstemperatur ca. 95°C.

### Beispiel 5: Hydroxylierung eines epoxidierten Sonnenblumenölfettsäure-diesters

Durch Veresterung von destillierter Sonnenblumenölfettsäure (Kennzahlen s. Beispiel 4) mit Ethylenglykol wurde ein Diester mit folgenden Kennzahlen hergestellt: SZ 6,3; JZ 131; Viskosität bei 25°C 40 mPa.s. Dieser Diester wurde nach bekanntem Verfahren mit Perameisensäure epoxidiert und besaß folgende Kennzahlen: SZ 6,3; JZ 1,1; Oxirangehalt 5,1 %; Viskosität bei 25°C 270 mPa.s. Dieser epoxidierte Diester wurde wie in Beispiel 1 beschrieben hydroxyliert.

Der Hydroxydiester besaß folgende Kennzahlen: SZ 29,4; JZ 9,2; Oxirangehalt 0,03 %; OHZ 261; Viskosität bei 25°C 2670 mPa.s.

### Beispiel 6: Hydroxylierung eines epoxidierten Rübölfettsäuremethylesters und anschließende Spaltung zur Hydroxyfettsäure

Epoxidierter Rübölfettsäure-methylester mit den Kennzahlen: SZ 0,9; JZ 1,4; Oxirangehalt 5,2 %; Viskosität bei 25°C 45 mPa.s wurde, wie in Beispiel 1 beschrieben, hydroxyliert. Der so hergestellte Hydroxyfettsäure-methylester besaß folgende Kennzahlen: SZ 9,2; JZ 11,2; Oxirangehalt 0,1 %; OHZ 282; Erweichungstemperatur ca. 45°C. Durch Verseifung dieses Hydroxyfettsäure-Methylesters mit alkoholischer Natronlauge und Spaltung der Seifen mit verdünnter Salzsäure konnte aus dem Hydroxyester die zugrundeliegende Hydroxyfettsäure isoliert werden.

Diese Hydroxyfettsäure hatte folgende Kennzahlen: SZ 175; VZ 184; JZ 12; OHZ 281; Erweichungstemperatur ca. 68°C.

### Beispiel 7: Hydroxylierung von epoxidiertem Rüböl in Gegenwart von säureaktiviertem Kieselgur

1000 g epoxidiertes Rüböl mit einem Oxirangehalt von 5,95 % wurden mit 100 g Wasser und 1 g eines mit 100 mval H₂SO₄/100 g aktivierten Kieselgurs (amorphe Kieselsäure vom Diatomeenerde-Typ) in einem Autoklaven unter Rühren auf 175°C erhitzt. Nach zweistündiger Reaktionszeit bei 175°C war der Oxirangehalt vollständig abgebaut.

Das hydroxylierte Rüböl besaß folgende Kennzahlen: SZ 19,8; JZ 13,5; Oxirangehalt 0,08 %, OHZ 197; Viskosität bei 25°C 13.700 mPa.s.

### Beispiel 8: Hydroxylierung von epoxidiertem Rüböl in Gegenwart einer säureaktivierten Aktivkohle

1000 g epoxidiertes Rüböl mit einem Oxirangehalt von 5,95 % wurde mit 100 g Wasser und 1 g einer mit 100 mval H₂SO₄/100 g aktivierten Aktivkohle in einem Autoklaven unter Rühren auf 175°C aufgeheizt und anschließend zwei Stunden bei dieser Temperatur gehalten. Die Reaktionszeit von zwei Stunden reichte aus, um einen vollständigen Abbau des Oxirangehaltes zu erreichen.

Das derart gewonnene hydroxylierte Rüböl besaß folgende Kennzahlen: SZ 15,4; JZ 13,3; Oxirangehalt 0,01 %; OHZ 214; Viskosität bei 25°C 29.000mPa.s.

### Beispiel 9: Hydroxylierung von epoxidiertem Rüböl in Gegenwart einer säureaktivierten Kieselsäure

1000 g epoxidiertes Rüböl mit einem Oxirangehalt von 5,95 % wurden mit 100 g Wasser und 1 g einer mit 100 mval H₂SO₄/100 g aktivierten Kieselsäure (SiO₂·XH₂O, kristallin) in einem Autoklaven unter Rühren auf ca. 175°C aufgeheizt. Nach einer Reaktionszeit von ca. zwei Stunden bei 175°C war die Hydroxylierungsreaktion bei vollständigem Abbau des Oxirangehalts abgeschlossen.

Das resultierende Produkt hatte folgende Kennzahlen: SZ 18,5; JZ 14,3; Oxirangehalt 0,01 %; OHZ 189; Viskosität bei 25°C 64.650 mPa.s.

Ein unter den oben beschriebenen Bedingungen durchgeführter Versuch mit einer nicht-aktivierten Kieselsäure führte zu keinem nennenswerten Abbau des Oxirangehaltes während der zweistündigen Reaktionsdauer bei 175°C. In diesem Fall war es also zu keiner Umwandlung von Oxiran- zu Hydroxylgruppen gekommen.

### Beispiel 10: Hydroxylierung von epoxidiertem Rüböl mit Ethanol

1000 g epoxidiertes Rüböl mit einem Oxirangehalt von 5,95 % wurden mit 200 g Ethanol und 1 g einer mit Schwefelsäure aktivierten Tonerde vom Montmorillonit-Typ in einem Autoklaven unter Rühren aufgeheizt. Bei etwa 165°C setzte die Reaktion unter Öffnung der Oxiranringe und Anlagerung von Ethanol ein und führte nach etwa 1 Stunden bei 180°C zu einem quantitativen Abbau des Oxirangehaltes.

Das derart gewonnene hydroxylierte Rüböl besaß folgende Kennzahlen: SZ 1,8; JZ 12; Oxirangehalt <0,1 %; OHZ 144; Viskosität bei 25°C 3240 mPa.s.

### Beispiel 11: Hydroxylierung von epoxidiertem Rüböl mit n-Butanol

Unter den gleichen Bedingungen wie in Beispiel 10 wurden 1000 g epoxidiertes Rüböl mit 277 g n-Butanol und 1 g säureaktivierter Tonerde umgesetzt.

Das so hergestellte hydroxylierte Rüböl besaß folgende Kennzahlen: SZ 1,8; JZ 13,5; Oxirangehalt <0,1 %; OHZ 193; Viskosität bei 25°C 1300 mPa.s.

### Beispiel 12: Hydroxylierung von epoxidiertem Rüböl mit C₈-/C₁₀-Alkohol

800 g epoxidiertes Rüböl wurden mit 432 g eines C₈-/C₁₀-Alkohols (Alfol 810-WZ) und 1 g einer mit Schwefelsäure aktivierten Tonerde in einem Autoklaven unter Rühren auf 200°C aufgeheizt. Nach einer Reaktionszeit von 2 Stunden bei 200°C war die Hydroxylierungsreaktion abgeschlossen.

Das resultierende Produkt hatte folgende Kennzahlen: SZ 1,8; JZ 15; Oxirangehalt <0,1 %; OHZ 153; Viskosität bei 25°C 425 mPa.s.

### Beispiel 13: Hydroxylierung von epoxidiertem Rüböl mit Monoethylenglykol

1000 g epoxidiertes Rüböl mit einem Oxirangehalt von 5,95 % wurden mit 230 g Monoethylenglykol und 1 g einer mit Schwefelsäure aktivierten Tonerde unter den in Beispiel 12 beschriebenen Bedingungen umgesetzt.

Die so gewonnene Polyhydroxyverbindung besaß folgende Kennzahlen: SZ 1,9; JZ 13,5; Oxirangehalt <0,1 %; OHZ 327; Viskosität bei 25°C 3400 mPa.s.

### Beispiel 14: Hydroxylierung von epoxidiertem Rüböl mit Polyglykol E 400

500 g epoxidiertes Rüböl mit einen Oxirangehalt von 5,95 % wurden mit 750 g Polyglykol E 400 und 1 g einer mit Schwefelsäure aktivierten Tonerde unter den in Beispiel 12 genannten Bedingungen umgesetzt.

Nach einer Reaktionszeit von 2 Stunden wurde ein hydroxyliertes Produkt mit folgenden Kennzahlen erhalten: SZ 2,0; JZ 16; Oxirangehalt <0,1 %; OHZ 189; Viskosität bei 25°C 350 mPa.s.

### Beispiel 15: Hydroxylierung von epoxidiertem Rüböl mit Diethanolamin

Eine Mischung aus 1000 g epoxidiertem Rüböl (Oxirangehalt 5,95 %), 390 g Diethanolamin und 1 g einer mit Schwefelsäure aktivierten Tonerde wurde in einem Autoklaven unter Rühren auf 180°C aufgeheizt.

Nach einer Reaktionszeit von 1,5 Stunden bei 180°C wurde eine Polyhydroxy-Verbindung mit folgenden Kennzahlen erhalten: SZ 2,2; JZ 11; Oxirangehalt <0,1 %; OHZ 462; Viskosität bei 25°C 24450 mPa.s.

## Patentansprüche

1. Verfahren zur Herstellung hydroxylierter Fettsäureverbindungen durch Oxidation epoxidierbarer Fettsäureverbindungen unter Ausbildung von Oxiranringen und säurekatalysierte Umwandlung der epoxidierten Fettsäureverbindungen, **dadurch gekennzeichnet,** daß man die einen oder mehrere mittelständige Oxiranringe aufweisenden epoxidierten Fettsäureverbindungen in Anwesenheit von säureaktivierten Tonerden und/oder Silikaten und/oder Aktivkohlen als Katalysator mit Wasser oder mit aktiven Wasserstoff enthaltenden Verbindungen ausgewählt aus der Gruppe von Alkoholen, Phenolen, Aminoalkoholen, Aminen, Mercaptanen, organischen Phosphorwasserstoff-Verbindungen (Phosphine), Carbonsäuren und Halogenwasserstoffen umsetzt.

2. Verfahren nach Anspruch 1, **dadurch gekennzeichnet,** daß man als Katalysator eine säureaktivierte Bleicherde vom Montmorillonit-Typ verwendet.

3. Verfahren nach einem oder mehreren der vorgehenden Ansprüche, **dadurch gekennzeichnet,** daß man für die Säureaktivierung des Katalysator-Trägermaterials Salzsäure und/oder andere mineralische und/oder organische Säuren, die zur Protonierung von Epoxidgruppen befähigt sind, verwendet.

4. Verfahren nach einem oder mehreren der vorhergehenden Ansprüche, **dadurch gekennzeichnet,** daß man epoxidierte Fettsäureverbindungen verwendet, bei denen die Oxiranringe im Bereich der Kohlenstoffatome 9 bis 16 je Fettsäurerest stehen.

5. Verfahren nach einem oder mehreren der vorhergehenden Ansprüche, **dadurch gekennzeichnet,** daß man als Ausgangsmaterial epoxidierbare pflanzliche und/oder tierische Öle und/oder Fettsäureverbindungen mit unterschiedlichen Anteilen an einfach und/oder mehrfach ungesättigten Fettsäuren verwendet.

6. Verfahren nach einem oder mehreren der vorhergehenden Ansprüche, **dadurch gekennzeichnet,** daß man den säureaktivierten Katalysator in einer Menge von 0,01 bis 0,1 Gew.-% der Fettsäureverbindung verwendet.

7. Verfahren nach einem oder mehreren der vorhergehenden Ansprüche, **dadurch gekennzeichnet,** daß man die epoxidierten Fettsäureverbindungen in einem geschlossenen System bei Temperaturen zwischen 80 und 200°C zur Reaktion bringt.

8. Verfahren nach einem oder mehreren der vorhergehenden Ansprüche, **dadurch gekennzeichnet,** daß man als Fettsäureverbindung epoxidiertes Rüböl, Euphorbiaöl und/oder Sonnenblumenöl verwendet.

## Claims

1. Process for the production of hydroxylated fatty acid compounds by oxidation of epoxidizable fatty acid compounds with the formation of oxirane rings and acid-catalyzed conversion of the epoxidized fatty acid compounds, **characterized in that** the epoxidized fatty acid compounds having one or more centrally located oxirane rings are reacted in the presence of acid-activated aluminas and/or silicates and/or activated charcoals as catalyst with water or with compounds containing active hydrogen selected from the group of alcohols, phenols, aminoalcohols, amines, mercaptans, organic hydrogen phosphide compounds (phosphines), carboxylic acids and hydrogen halides.

2. Process according to Claim 1, **characterized in that** an acid-activated bleaching earth of the montmorillonite type is used as catalyst.

3. Process according to one or more of the preceding claims, **characterized in that** hydrochloric acid and/or other mineral and/or organic acids which are capable of protonating epoxide groups are used for the acid activation of the catalyst support material.

4. Process according to one or more of the preceding claims, **characterized in that** epoxidized fatty acid compounds are used in which the oxirane rings stand in the region of carbon atoms 9 to 16 per fatty acid group.

5. Process according to one or more of the preceding claims, **characterized in that** epoxidizable vegetable and/or animal oils and/or fatty acid compounds with varying amounts of mono- and/or polyunsaturated fatty acids are used as starting material.

6. Process according to one or more of the preceding claims, **characterized in that** the acid-activated catalyst is used in a quantity of 0.01 to 0.1 % by wt. of the fatty acid compound.

7. Process according to one or more of the preceding claims, **characterized in that** the epoxidized fatty acid compounds are reacted in a closed system at temperatures between 80 and 200°C.

8. Process according to one or more of the preceding claims, **characterized in that** epoxidized rapeseed oil, euphorbia oil and/or sunflower oil is used as fatty acid compound.

## Revendications

1. Procédé pour la préparation de composés de type acide gras hydroxylés, par oxydation de composés de type acide gras époxydables, avec formation de cycles oxiranne et conversion, catalysée par un acide, des composés de type acide gras époxydés, caractérisé en ce que l'on fait réagir les composés de type acide gras époxydés, comportant un ou plusieurs cycles oxiranne en milieu de chaîne, en présence, en tant que catalyseur, d'alumine et/ou de silicates et/ou de charbon actif, activés par un acide, avec de l'eau ou des composés contenant de l'hydrogène actif, choisis parmi des alcools, phénols, aminoalcools, amines, mercaptans, composés hydrogénophosphorés (phosphines), acides carboxyliques et hydracides halogénés.

2. Procédé selon la revendication 1, caractérisé en ce que l'on utilise comme catalyseur une terre décolorante du type montmorillonite, activée par un acide.

3. Procédé selon une ou plusieurs des revendications précédentes, caractérisé en ce que, pour l'activation par un acide du matériau de support-catalyseur, on utilise l'acide chlorhydrique et/ou d'autres acides organiques et/ou acides minéraux, qui sont aptes à la protonation de groupes époxy.

4. Procédé selon une ou plusieurs des revendications précédentes, caractérisé en ce que l'on utilise des composés de type acide gras époxydés dans lesquels les cycles oxiranne se trouvent dans la région des atomes de carbone 9 à 16 de chaque radical acide gras.

5. Procédé selon une ou plusieurs des revendications précédentes, caractérisé en ce que, comme produit de départ, on utilise des huiles minérales et/ou des huiles végétales et/ou des composés de type acide gras comportant diverses fractions d'acides gras à une et/ou plusieurs insaturations, aptes à l'époxydation.

6. Procédé selon une ou plusieurs des revendications précédentes, caractérisé en ce que l'on utilise le catalyseur activé par un acide en une proportion de 0,01 à 0,1 % en poids, par rapport au composé de type acide gras.

7. Procédé selon une ou plusieurs des revendications précédentes, caractérisé en ce que l'on met en réaction les composés de type acide gras époxydés, dans un système clos, à des températures comprises entre 80 et 200°C.

8. Procédé selon une ou plusieurs des revendications précédentes, caractérisé en ce que, en tant que composé de type acide gras, on utilise de l'huile de betterave, de l'huile d'euphorbe et/ou de l'huile de tournesol époxydées.
